# EUROPEAN PATENT APPLICATION

(11) **EP 1 484 317 A1**
(43) Date of publication of application: **08.12.2004**
(21) Application number: 03705269.3
(22) Date of filing: 18.02.2003
(51) Int. Cl.: C07C 271/22, C07C 269/06, C07D 213/70, C07D 309/12

(54) **LACTIC ACID DERIVATIVE**

(30) Priority: 19.02.2002 JP 2002042010
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); MURAKAMI, Masahiro, Osaka-shi, Osaka 547-0026 (JP)
(74) Representative: Sternagel, Fleischer, Godemeyer & Partner
(86) International application number: PCT/JP2003/001698
(87) International publication number: WO 2003/070693

(57) **Abstract**

An object of the present invention is to provide a lactic acid derivative having an aminoethyl group at the terminus and a method for producing the same. According to the present invention, there is provided a compound represented by formula (1) or a salt thereof: 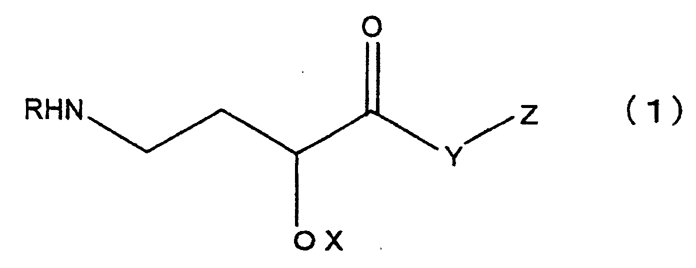 wherein R represents a hydrogen atom or a protective group for an amino group; X represents a hydrogen atom or a protective group for a hydroxyl group; Y represents -O-or -S-; and Z represents an aliphatic group, an aryl group, a heterocyclic group, a -CO- group, or a combination thereof, which may have a substituent.

## Description

### TECHNICAL FIELD

The present invention relates to a lactic acid derivative, and a method for producing the same. More particularly, the present invention relates to a lactic acid derivative having an aminoethyl group at the terminus, and a method for producing the same.

### BACKGROUND ART

A mixture of cyclic and/or linear poly L-lactic acids having condensation degrees of 3-19 is reported to be useful as an anti-malignant tumor agent (Japanese Patent Laid-open Publication Nos. 9-227388 and 10-130153) and a QOL improving agent for a cancer patient (JP Application No. 11-39894; Journal of Japan Society of Clinical Oncology, vol. 33, No. 3, p493). Furthermore, a mixture of cyclic and/or linear poly L-lactic acids having condensation degree of 3-19 is known to have a blood sugar reducing action so that it is useful as a medicament for prevention and/or treatment of diabetes or a diabetic complication (JP Application No. 11-224883), and is further proved to be useful for suppressing over-appetite, promoting basal metabolism, and improving and/or preventing obesity.

As described above, the mixture of cyclic and/or linear poly L-lactic acids having condensation degree of 3-19 has been demonstrated to exhibit various pharmacological effects, and is expected to be developed as a medicament in future. In order to develop an oligolactic acid as a medicament, it is thought that not only use of a lactic acid, but also use of various types of lactic acid derivatives as a constitutional unit will be useful.

### DISCLOSURE OF THE INVENTION

A problem to be solved by the present invention is to provide a lactic acid derivative having an aminoethyl group at the terminus and a method for producing the same.

The present inventors made diligent studies to solve the above problem, and as a result, they have succeeded in synthesizing a novel lactic acid derivative by using a lactic acid having an aminoethyl group at the terminus as a starting material and thioesterifying or esterifying it. The present invention has been completed based on this fmding. A lactic acid derivative of the present invention can be used to produce a lactic acid oligomer. Such the lactic acid oligomer is expected to exert various physiological actions and is useful for developing various medicaments.

Namely, the present invention provides a compound represented by formula (1) or a salt thereof: 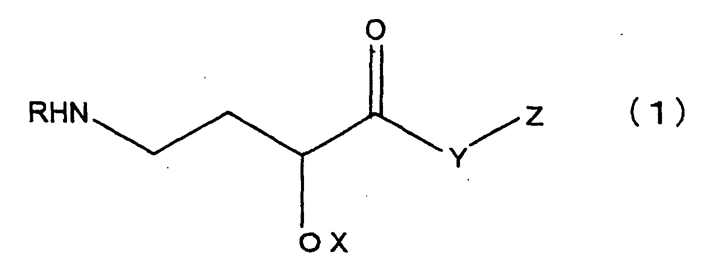 wherein R represents a hydrogen atom or a protective group for an amino group; X represents a hydrogen atom or a protective group for a hydroxyl group; Y represents -O-or -S-; and Z represents an aliphatic group, an aryl group, a heterocyclic group, a -CO- group, or a combination thereof, which may have a substituent.

Preferably, Z is a pyridyl group, or Z is a phenacyl group which may have a substituent.

Another aspect of the present invention provides a method for producing a compound represented by formula (1): 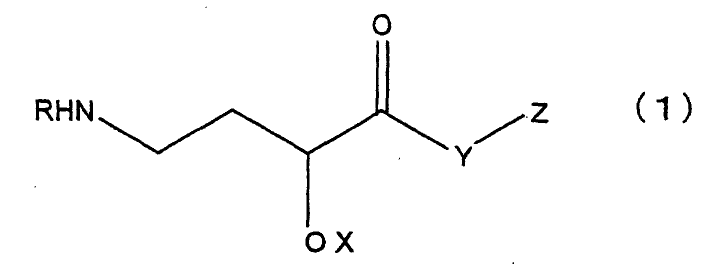 wherein R represents a hydrogen atom or a protective group for an amino group; X represents a hydrogen atom or a protective group for a hydroxyl group; Y represents -S-; and Z represents an aliphatic group, an aryl group, a heterocyclic group, a -CO- group, or a combination thereof, which may have a substituent,
which comprises reacting a compound represented by formula (2): 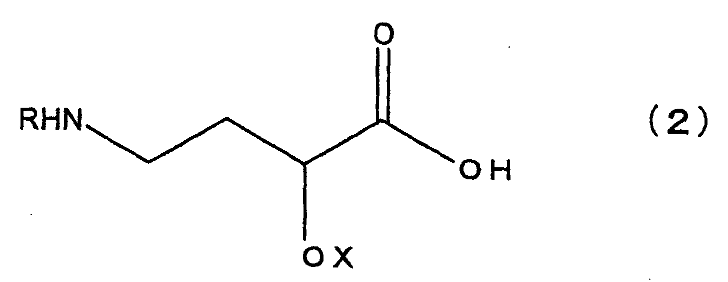 wherein R represents a hydrogen atom or a protective group for an amino group; and X represents a hydrogen atom or a protective group for a hydroxyl group,
with a compound represented by a formula Z-S-S-Z wherein Z represents an aliphatic group, an aryl group, a heterocyclic group, a -CO- group, or a combination thereof, which may have a substituent.

Further another aspect of the present invention provides a method for producing a compound represented by formula (1): 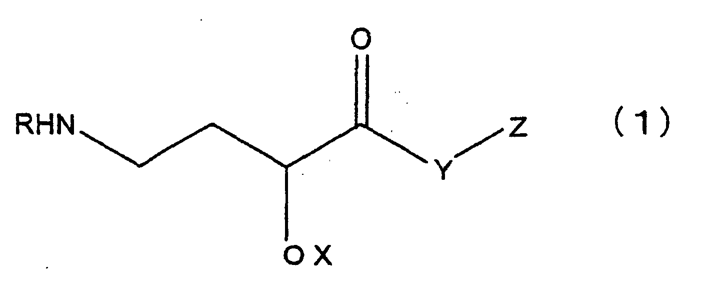 wherein R represents a hydrogen atom or a protective group for an amino group; X represents a hydrogen atom or a protective group for a hydroxyl group; Y represents -O-; and Z represents an aliphatic group, an aryl group, a heterocyclic group, a -CO- group, or a combination thereof, which may have a substituent,
which comprises reacting a compound represented by formula (2): 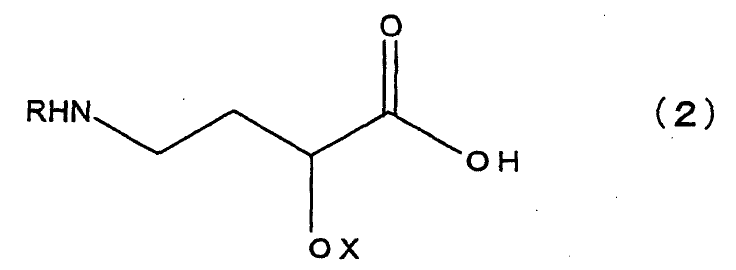 wherein R represents a hydrogen atom or a protective group for an amino group; and X represents a hydrogen atom or a protective group for a hydroxyl group,
with a compound represented by a formula Br-Z wherein Z represents an aliphatic group, an aryl group, a heterocyclic group, a -CO- group, or a combination thereof, which may have a substituent.

### MODE FOR CARRYING OUT THE INVENTION

Embodiments and practice methods of the present invention will be described in detail below.

The present invention relates to a compound represented by a formula (1) or a salt thereof: 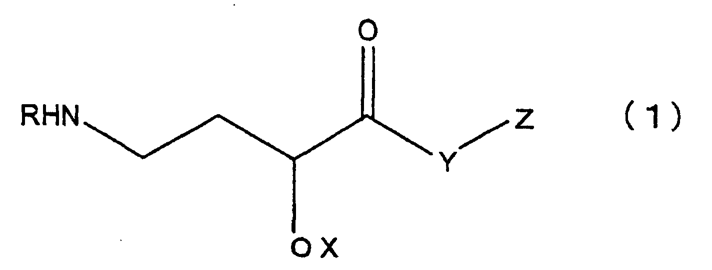 wherein R represents a hydrogen atom or a protective group for an amino group; X represents a hydrogen atom or a protective group for a hydroxyl group; Y represents -O- or -S-; and Z represents an aliphatic group, an aryl group, a heterocyclic group, a -CO- group, or a combination thereof, which may have a substituent.

The protective group for an amino group represented by R is not particularly limited, and may be suitably selected by a person skilled in the art. Specific examples of the protective group for an amino group include formyl group, C₁₋₆ alkyl carbonyl group (such as acetyl or ethylcarbonyl), C₁₋₆ alkyl sulfonyl group, tert-butyloxycarbonyl group (hereinafter, also called Boc), benzyloxycarbonyl group, allyloxycarbonyl group, fluorenylmethyloxycarbonyl group, arylcarbonyl group (such as phenylcarbonyl or naththylcarbonyl), arylsulfonyl group (such as phenylsulfonyl or naththylsulfonyl), C₁₋₆ alkyloxycarbonyl group (such as methoxycarbonyl or ethoxycarbonyl), C₇₋₁₀ aralkylcarbonyl group (such as benzylcarbonyl), methyl group, and aralkyl group (such as benzyl, diphenylmethyl, or trityl). These groups may be substituted with one to three halogen atoms (such as fluorine, chlorine, or bromine) or nitro groups. Specific examples thereof include p-nitrobenzyloxycarbonyl group, p-chlorobenzyloxycarbonyl group, m-chlorobenzyloxycarbonyl group, and p-methoxybenzyloxycarbonyl group. Specific examples of particularly preferable protective group for an amino group include benzyloxycarbonyl group and tert-butyloxycarbonyl group.

The protective group for a hydroxyl group represented by X is not particularly limited, and may be suitably selected by a person skilled in the art. Specific examples of the protective group for a hydroxyl group include those described below:
(Ether type)
   methyl group, methoxymethyl group, methylthiomethyl group, benzyloxymethyl group, t-butoxymethyl group, 2-methoxyethoxymethyl group, 2,2,2-trichloroethoxymethyl group, bis(2-chloroethoxy)methyl group, 2-(trimethylsilyl)ethoxymethyl group, tetrahydropyranyl group, 3-bromotetrahydropyranyl group, tetrahydrothiopyranyl group, 4-methoxytetrahydropyranyl group, 4-methoxytetrahydrothiopyranyl group, 4-methoxytetrahydrothiopyranyl S,S-dioxide group, tetrahydrofuranyl group, tetrahydrothiofuranyl group;
   1-ethoxyethyl group, 1-methyl-1-methoxyethyl group, 1-(isopropoxy)ethyl group, 2,2,2-trichloroethyl group, 2-(phenylserenyl)ethyl group, t-butyl group, allyl group, cinnamyl group, p-chlorophenyl group, benzyl group, p-methoxybenzyl group, o-nitrobenzyl group, p-nitrobenzyl group, p-halobenzyl group, p-cyanobenzyl group, 3-methyl-2-picolyl N-oxide group, diphenylmethyl group, 5-dibenzosuberyl group, triphenylmethyl group, a-naphthyldiphenylmethyl group, p-methoxyphenyldiphenylmethyl group, p-(p'-bromophenacyloxy)phenyldiphenylmethyl group, 9-anthryl group, 9-(9-phenyl)xanthenyl group, 9-(9-phenyl-10-oxo)anthryl group, and benzisothiazolyl S,S-dioxide group;
   trimethylsilyl group, triethylsilyl group, isopropyldimethylsilyl group, t-butyldimethylsilyl group (TBDMS group), (triphenylmethyl)dimethylsilyl group, t-butyldiphenylsilyl group, methyldiisopropylsilyl group, methyl di-t-butylsilyl group, tribenzylsilyl group, tri-p-xylylsilyl groupl, triisopropylsilyl group, and triphenylsilyl group;
(Ester type)
   formate, benzoyl formate, acetate, chloroacetate, dichloroacetate, trichloroacetate, trifluoroacetate, methoxyacetate, triphenylmethoxyacetate, phenoxyacetate, p-chlorophenoxyacetate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, p-P-phenylacetate, 3-phenylpropionate, 3-benzoylpropionate, isobutylate, monosuccinoate, 4-oxopentanoate, pivaloate, adamantoate, crotonate, 4-methoxycrotonate, (E)-2-methyl-2-butenoate, benzoate, o-(dibromomethyl)benzoate, o-(methoxycarbonyl)benzoate, p-phenylbenzoate, 2,4,6-trimethylbenzoate, p-P-benzoate, and a-naphthoate;
(Carbonate type)
   methylcarbonate, ethylcarbonate, 2,2,2-trichloroethylcarbonate, isobutylcarbonate, vinylcarbonate, allylcarbonate, cinnamylcarbonate, p-nitrophenylcarbonate, benzylcarbonate, p-methoxybenzylcarbonate, 3,4-dimethoxybenzylcarbonate, o-nitrobenzylcarbonate, p-nitrobenzylcarbonate, and S-benzylthiocarbonate; and
(others)
   N-phenylcarbamate, N-imidazolylcarbamate, borate, nitrate, N,N,N',N'-tetramethylphosphorodiamidate, and 2,4-dinitrophenylsulfenate.

A method for introducing and removing a protective group for amino group or hydroxyl group as described above are known to a person skilled in the art, and described in, for example, Teodora, W. Green, Protective Groups in Organic Synthesis, John & Wiley & Sons Inc. (1981) etc.

In the present invention, Z represents an aliphatic group, an aryl group, a heterocyclic group, a -CO- group, or a combination thereof, and they may have a substituent.

The aliphatic group, the aryl group or the heterocyclic group represented by Z are not particularly limited.

The aliphatic group in the present invention includes a lower alkyl group, a lower alkenyl group, and a lower alkynyl group. The carbon number of the aliphatic group is not particularly limited, but generally 1-10, preferably 1-6, and more preferably 1-4. The chain type of the aliphatic group is not particularly limited, and may be linear, branched, cyclic, or of a combination of these.

For the aliphatic group in the present invention, a lower alkyl group is particularly preferable. Specific examples of the lower alkyl group include methyl group, ethyl group, propyl group, isopropyl group, cyclopropyl group, butyl group, isobutyl group, sec-butyl group, tert-butyl group, cyclopropylmethyl group, cyclobutyl group, pentyl group and hexyl group.

For the aryl group in the present invention, an aryl group having a carbon number of 6-24, preferably 6-12 is suitable, and the aryl group may have one or more substituents. Specific examples of the aryl group include phenyl, naphthyl and p-methoxyphenyl.

For the heterocyclic group in the present invention, a saturate or unsaturated 5-10 membered monocyclic or fused cyclic group containing one or more oxygen atoms, nitrogen atoms or sulphur atoms is suitable. Specific examples of the heterocyclic group include pyridyl, imidazolyl, quinolyl, isoquinolyl, pyrimidinyl, pyrazinyl, pyridazinyl, phthalazinyl, triazinyl, furyl, thienyl, pyrrolyl, oxazolyl, thiazolyl, thiadiazolyl, triazolyl, benzimidazolyl, pyrrolydino and morphorino. These heterocyclic groups may have one or more substituents.

Examples of the substituent, which the aryl group or the heterocyclic group may have, include a halogen atom (fluorine, chlorine, bromine or iodine), alkyl group, aryl group, carbonamide group, alkylsulfonamide group, arylsulfonamide group, alkoxy group, aryloxy group, alkylthio group, arylthio group, carbamoyl group, sulfamoyl group, cyano group, alkylsulfonyl group, arylsulfonyl group, alkoxycarbonyl group, aryloxycarbonyl group and acyl group.

In the present invention, Z is particularly preferably a pyridyl group, or a phenacyl group which may have a substituent (such as p-bromophenacyl group).

When X is a hydrogen atom in the formula (1), the compound of the present invention can exist also as a metal salt. Examples of the metal salt include alkali metal salts such as sodium salt and potassium salt, alkali earth metal salts such as magnesium salt and calcium salt, aluminum salt, and zinc salt.

When R is a hydrogen atom in the formula (1), the compound of the present invention can exist as an acid addition salt. Specific examples of the acid addition salt include an inorganic acid salt such as hydrochloric acid salt, sulfuric acid salt, nitric acid salt and phosphoric acid salt, and an organic acid salt such as acetic acid salt, maleic acid salt, fumaric acid salt and citric acid salt.

In addition, various types of hydrates, solvates and crystal forms of the compound of the present invention are included within the present invention.

Since the compound of the present invention contains an asymmetric carbon, there are stereoisomers. All the possible isomers and a mixture containing two or more types of the isomers at an arbitrary ratio are also included within the present invention. Therefore, the compound of the present invention includes a mixture of various optical isomers such as an optically active substance, a racemic body and a diastereomer, and the isolated single substances thereof.

The configuration of the compound of the present invention depends on that of a lactic acid unit in a compound used as the starting material. That is, use of the L form, the D form, or a mixture thereof as the lactic acid unit in a starting material compound gives the compound of the invention in various configurations. In the present invention, as the configuration of the lactic acid unit, the L form is used preferably.

The preferable configuration of the compound of the present invention is represented by formula (3) below:

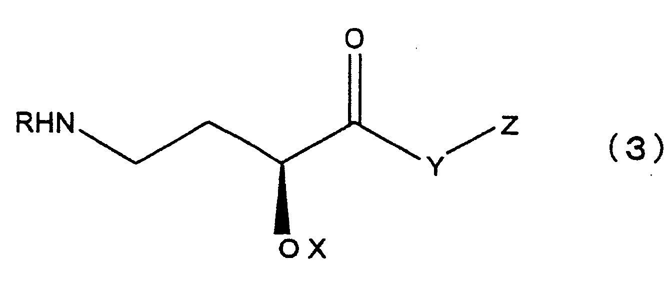

In the formula (3), R, X, Y and Z have the same meanings as defined in the formula (1).

Next, the method for producing the compound of the present invention will be described.

The compound of the formula (1) wherein Y is -S-, can be produced by reacting the compound represented by the formula (2): 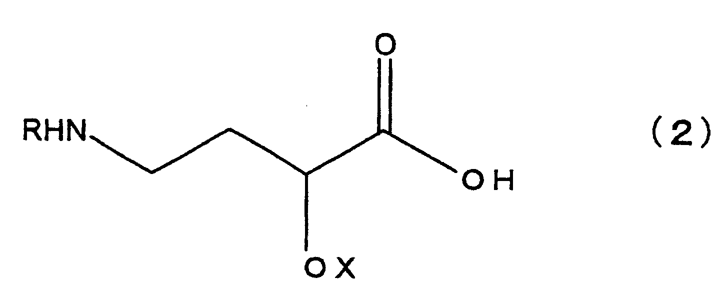 wherein R represents a hydrogen atom or a protective group for an amino group; and X represents a hydrogen atom or a protective group for a hydroxyl group, with a compound represented by a formula Z-S-S-Z wherein Z represents an aliphatic group, an aryl group, a heterocyclic group, a -CO- group, or a combination thereof, which may have a substituent.

In the case where the compound represented by the formula (2) is reacted with the compound represented by the formula Z-S-S-Z, reaction can be carried out by mixing together a solution containing the compound represented by the formula (2), a triphenylphosphine solution, and a solution containing the compound represented by the formula Z-S-S-Z, followed by stirring for a certain time.

Amounts of the compound represented by the formula (2) and the compound represented by the formula Z-S-S-Z to be used can be selected suitably, but it ranges preferably from 1:0.7 to 1:2, more preferably from 1:1 to 1:1.7.

The reaction temperature is not particularly limited, as long as the reaction proceeds, but is preferably -100 °C to room temperature.

The reaction is preferably carried out in the presence of a reaction solvent. The reaction solvent is not particularly limited, as long as it is inactive to the reaction, but, preferably, toluene, benzene, xylene, alkylbenzene, tetrahydrofuran (THF), diethyl ether, or dimethoxyethane can be used.

For the reaction atmosphere, an inert gas atmosphere such as nitrogen gas or argon gas can be used.

One of the preferable embodiments of the reaction described above will be described specifically below.

A solution of the compound represented by the formula Z-S-S-Z (for example, 2,2'-dipyridyldisulfide) in toluene, a solution of triphenylphosphine in toluene and a solution of the compound represented by the formula (2) (for example, N-Boc-(S)-(-)-4-amino-2-hydroxybutanoic acid) in THF are put in an argon-substituted flask, followed by stirring for several hours. After removing THF in a reduced pressure, water is added to stop the reaction. Then, by ordinary purification and isolation, the compound of the formula (1) wherein Y is -S- can be obtained.

The compound of the formula (1) wherein Y is -O- can be produced by reacting a compound represented by the formula (2): 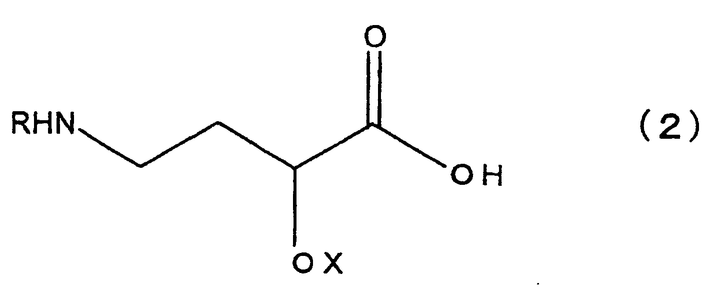 wherein R represents a hydrogen atom or a protective group for an amino group; and X represents a hydrogen atom or a protective group for a hydroxyl group,
with a compound represented by the formula Br-Z wherein Z represents an aliphatic group, an aryl group, a heterocyclic group, a -CO- group, or a combination thereof, which may have a substituent.

In the case where the compound represented by the formula (2) is reacted with the compound represented by the formula Br-Z, reaction can be carried out by mixing the compound represented by the formula (2) and the compound represented by the formula Br-Z in a suitable solvent in the presence of sodium hydrogen carbonate, followed by stirring for a certain time.

Amounts of the compound represented by the formula (2) and the compound represented by the formula Br-Z to be used can be selected suitably, but it ranges preferably from 1:0.7 to 1:2, more preferably from 1:1 to 1:1.7.

The reaction temperature is not particularly limited as long as the reaction proceeds, but is preferably -100 °C to room temperature.

The reaction is preferably carried out in the presence of a reaction solvent. The reaction solvent is not particularly limited as long as it is inactive to the reaction, but, preferably, ethanol/acetone or the like can be used.

For the reaction atmosphere, an inert gas atmosphere such as nitrogen gas or argon gas can be used.

One of the preferable embodiments of the reaction described above will be described specifically below.

Sodium hydrogen carbonate, ethanol, and a solution of the compound represented by the formula (2) (for example, N-Boc-(S)-(-)-4-amino-2-hydroxybutanoic acid) in acetone are put in an argon-substituted flask, followed by stirring. A solution of the compound represented by the formula Br-Z (for example, p-bromophenacylbromide) in acetone is added thereto, followed by stirring for an additional certain time. After the reaction, by ordinary purification and isolation, a compound of the formula (1) wherein Y is -O- can be obtained.

The present invention will be described in more detail by the following examples, but the present invention is not limited to the examples.

### EXAMPLES

### Example 1: Production of N-Boc-(S)-(-)-4-amino-2-hydroxybutanoic acid 2-pyridyl thiol ester (esterification by pyridine thiol)

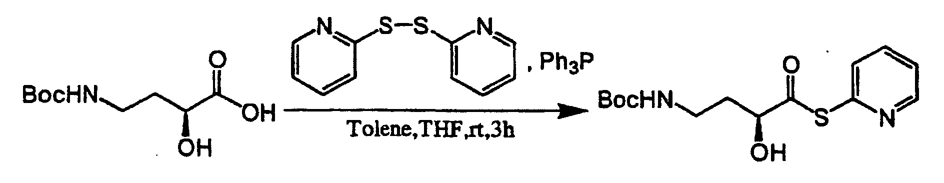

A solution of 2,2-pyridyldisulfide (2.5777 g) (11.7 mmol) in toluene (10 ml), a solution of triphenylphosphine (3.0688 g) (11.7 mmol) in toluene (20 ml), and a solution of N-Boc-(S)-(-)-4-amino-2-hydroxybutanoic acid (1.7103 g) (7.8 mmol) in THF (4 ml) were put in an argon-substituted two-neck eggplant-shaped flask, followed by stirring for 1.5 hours. After removing THF in a reduced pressure, water was added to stop the reaction. The resultant was extracted three times with chloroform, dried over magnesium sulfate, and concentrated to give a product (7.618 g), which was separated by silica gel column chromatography using an ether:chloroform=1:1 solution to obtain N-Boc-(S)-(-)-4-amino-2-hydroxybutanoic acid 2-pyridyl thiol ester at a yield of 0.9793 g (3.13 mmol, 40%).
H NMR (300MHz, CDCl₃)
d (ppm)=1.38 (9H, s)
1.85-2.03 (1H, m)
2.21-2.34 (1H, m)
2.33 (2H, dd, J=5.4, 11.4Hz)
4.02 (1H, t, J=7.2Hz)
4.89 (1H, bs)
7.21 (1H, ddd, J=2.7, 5.1, 7.8Hz)
6.8 (1H, dt, J=1.8, 7.8Hz)
8.42 (1H, d, J=2.7Hz)

### Example 2: Production of N-Boc-(S)-(-)-4-amino-2-hydroxybutanoic acid p-bromophenacyl and its protection

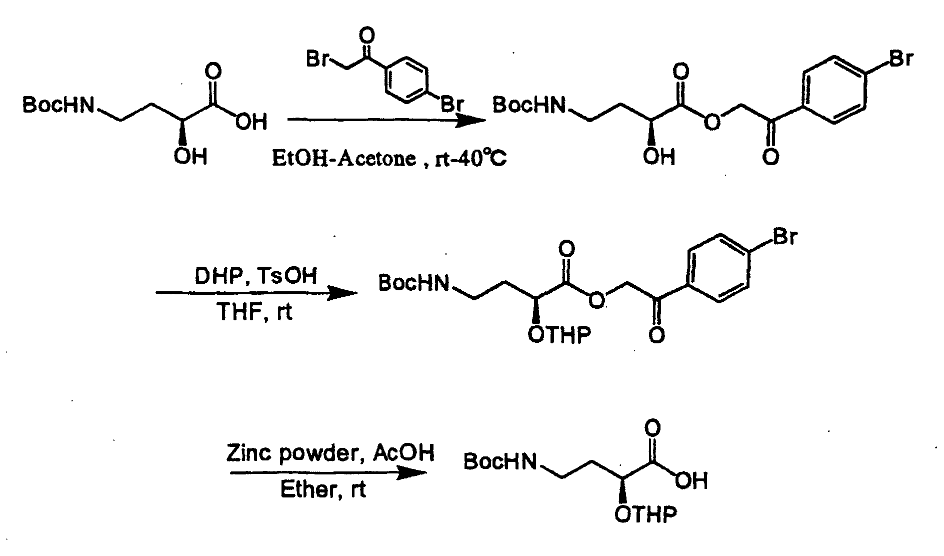

### (1) Esterification by p-bromophenacyl

Sodium hydrogen carbonate (0.4221 g) (5.0 mmol), ethanol (15 ml), and a solution of N-Boc-(S)-(-)-4-amino-2-hydroxybutanoic acid (1.1054 g) (5.0 mmol) in acetone (6 ml) were put in a 100 ml argon-substituted two-neck eggplant-shaped flask, followed by stirring for 10 minutes. A solution of p-bromophenacylbromide (2,4'-dibromoacetophenone) in acetone 25 ml was added, followed by stirring for additional 11 hours. An aqueous saturated sodium hydrogen carbonate solution (20 ml) was added thereto. The resultant was extracted three times with chloroform (50 ml), dried over magnesium sulfate, and concentrated to give a product (1.9075 g). The product was separated by silica gel column chromatography using an ether:hexane=1:1 solution to obtain N-Boc-(S)-(-)-4-amino-2-hydroxybutanoic acid p-bromophenacyl at a yield of 1.5831 g (3.8 mmol, 75%).
H NMR (300MHz, CDCl₃)
d (ppm)=1.43 (9H, s)
1.96-2.07 (1H, m)
2.11-2.22 (1H, m)
3.34-3.47 (2H,m)
3.55 (1H, d, J=5.4Hz)
4.44-4.50 (1H, m)
5.00 (1H, bs)
5.32 (1H, d, J=16Hz)
5.50 (1H, d, J=16Hz)
7.66 (2H, d, J=8.4Hz)
7.78 (2H, d, J=8.4Hz)

### (2) Etherification by THP

A solution of N-Boc-(S)-(-)-4-amino-2-hydroxybutanoic acid p-bromophenacyl (0.8245 g) (2.0 mmol) in THF (7 ml), a catalytic amount of p-toluene sulfonic acid, and a solution of dihydropyrane (0.3349 g) (4.0 mmol) in THF (3 ml) were put in a 100ml argon-substituted two-neck eggplant-shaped flask, followed by stirring for 2.5 hours. An aqueous saturated sodium chloride solution (20 ml) was added thereto. The resultant was extracted three times with chloroform (50 ml), dried over magnesium sulfate, and concentrated to give a product (1.4849 g). The product was separated by silica gel column chromatography using a solution of ether:hexane=4:1 to obtain N-Boc-O-(2-tetrahydropyranyl)-(S)-(-)-4-amino-2-hydroxybutanoic acid p-bromophenacyl at a yield of 0.8910 g (1.7 mmol, 90%).
H NMR (300MHz, CDCl3)
d (ppm)=0.84 (2H, t, J=6.6Hz)
1.41 (9H, s)
1.15-2.19 (7H, m)
3.20-3.48 (2H, m)
3.52-3.56 (1H,m)
3.80-3.98 (1H, m)
4.57 (1H, dd, J=4.5, 6.9Hz)
4.68-4.73 (1H, m )
5.21-5.43 (2H, m)
7.60 (2H, d, J=8.4Hz)
7.74 (2H, d, J=8.4Hz)

### (3) Deesterification of p-bromophenacyl

Acetic acid (2.4760 g) (41.2 mmol) and zinc powder (2.6887 g) (41.1 mmol) were put in a 100ml argon-substituted two-neck eggplant-shaped flask, followed by stirring for 10 minutes. N-Boc-O-THP-(S)-(-)-4-amino-2-hydroxybutanoic acid p-bromophenacyl ester (0.6876 g) (1.4 mmol) was added thereto. The resultant was washed with ether (48 ml), stirred for 3 hours, and filtered so as not to dry the zinc powder. An aqueous saturated sodium hydrogen carbonate solution (30 ml) was added thereto. The resultant was extracted three times with ether (50 ml), dried over magnesium sulfate, and concentrated to give a product (0.1152 g). The product was separated by silica gel column chromatography using an ether solution to obtain N-Boc-O-(2-tetrahydropyranyl)-(S)-(-)-4-amino-2-hydroxybutanoic acid at a yield of 0.3544 g (1.2 mmol, 85%).
H NMR (300MHz, CDCl₃)
d (ppm)=1.38 (9H, s)
1.50-1.94 (8H, m)
3.09-3.50 (3H, m)
3.76-3.83 (1H, m)
3.93-4.00 (1H, m)
4.31 (1H, dd, J=3.9, 8.7Hz)

### INDUSTRIAL APPLICABILITY

The present invention makes it possible to provide a lactic acid derivative having an aminoethyl group at the terminus. The lactic acid derivative of the present invention is a novel compound. By producing an oligolactic acid derivative by using the lactic acid derivative of the present invention, it becomes possible to develop a drug, a drug raw material, a food additive, a cosmetic raw material, a pharmaceutic raw material, and a pharmaceutic additive.

## Claims

1. A compound represented by formula (1) or a salt thereof: 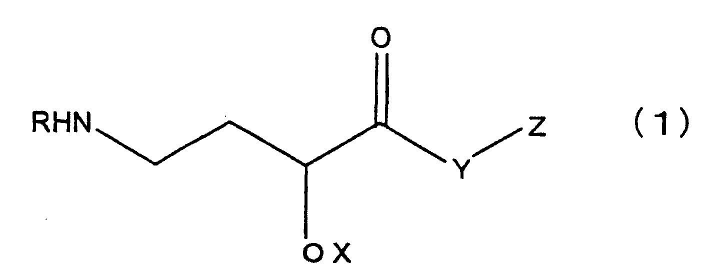 wherein R represents a hydrogen atom or a protective group for an amino group; X represents a hydrogen atom or a protective group for a hydroxyl group; Y represents -O- or -S-; and Z represents an aliphatic group, an aryl group, a heterocyclic group, a -CO- group, or a combination thereof, which may have a substituent.

2. The compound according to claim 1 or a salt thereof wherein Z is a pyridyl group.

3. The compound according to claim 1 or a salt thereof wherein Z is a phenacyl group which may have a substituent.

4. A method for producing a compound represented by formula (1): 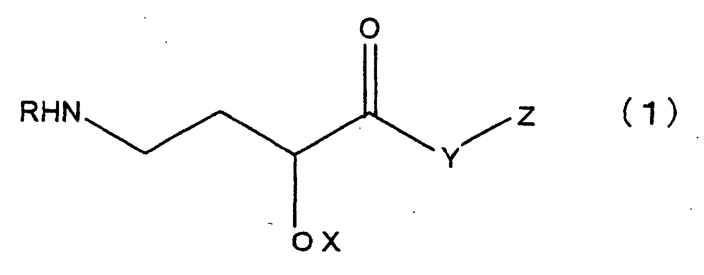 wherein R represents a hydrogen atom or a protective group for an amino group; X represents a hydrogen atom or a protective group for a hydroxyl group; Y represents -S-; and Z represents an aliphatic group, an aryl group, a heterocyclic group, a -CO- group, or a combination thereof, which may have a substituent,
which comprises reacting a compound represented by formula (2): 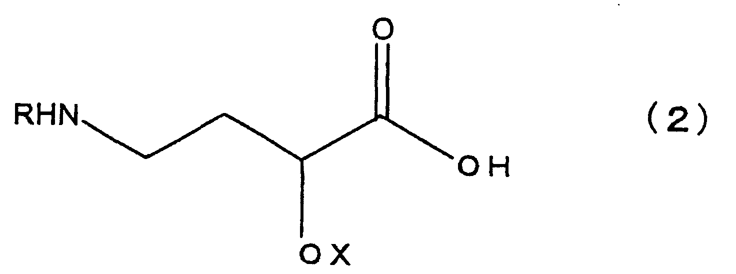 wherein R represents a hydrogen atom or a protective group for an amino group; and X represents a hydrogen atom or a protective group for a hydroxyl group,
with a compound represented by a formula Z-S-S-Z wherein Z represents an aliphatic group, an aryl group, a heterocyclic group, a -CO- group, or a combination thereof, which may have a substituent.

5. A method for producing a compound represented by formula (1): 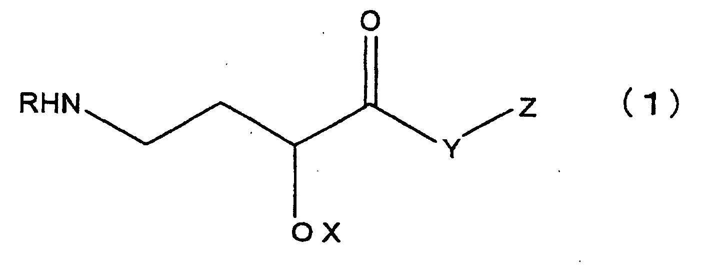 wherein R represents a hydrogen atom or a protective group for an amino group; X represents a hydrogen atom or a protective group for a hydroxyl group; Y represents -O-; and Z represents an aliphatic group, an aryl group, a heterocyclic group, a -CO- group, or a combination thereof, which may have a substituent,
which comprises reacting a compound represented by formula (2): 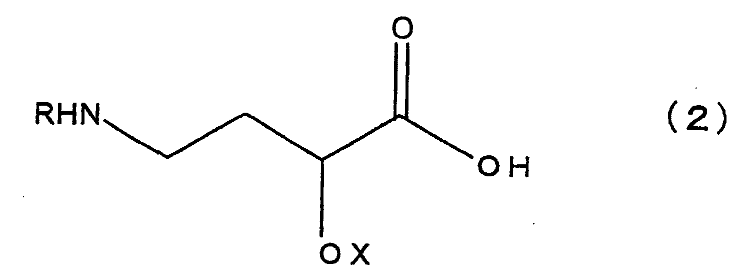 wherein R represents a hydrogen atom or a protective group for an amino group; and X represents a hydrogen atom or a protective group for a hydroxyl group,
with a compound represented by a formula Br-Z wherein Z represents an aliphatic group, an aryl group, a heterocyclic group, a -CO- group, or a combination thereof, which may have a substituent.
